# EUROPEAN PATENT APPLICATION

(11) **EP 2 422 763 A1**
(43) Date of publication of application: **29.02.2012**
(21) Application number: 10382241.7
(22) Date of filing: 27.08.2010
(51) Int. Cl.: A61K 8/65, A61K 8/44, A61Q 5/12

(54) **Process and kit for treating hair**

(71) Applicant: Colomer Beauty and Professional Products, S.L., 08940 Cornellà de Llobregat- Barcelona (ES)
(72) Inventor: Bermúdez Vico, Manuela, E-08940, Cornellà de Llobregat - Barcelona (ES); Delpani, Lorenzo, E-08940, Cornellà de Llobregat - Barcelona (ES)
(74) Representative: ABG Patentes, S.L.

(57) **Abstract**

A process for improving, among other properties, the resistance of hair to break and a kit specially adapted to carry out such process.

## Description

### FIELD OF THE INVENTION

The present invention relates to a process for improving, among other properties, the resistance of hair to break and a kit specially adapted to carry out such process.

### BACKGROUND

It is well known that the hair is made sensitive or fragile to various degrees by a variety of stresses. Among these stressing factors we can mention some environmental agents such as light, as well as the repeated action of various hair treatments such as shampooing, rinsing, drying, heating, combing, styling (such as permanent waving, relaxing or straightening), dyeing and bleaching.

Such stresses can cause a number of changes in hair leaving it in a dry, rough, lusterless, or frizzy condition, which can be caused, e.g., by repeated abrasion of the hair surface and removal of the hair's natural oils and other natural conditioning and moisturizing components. Additionally, hair is often subjected to weather-related stresses, e.g., sunlight, wind, and changes in temperature and humidity, which can cause hair damaged and other conditions considered by consumers to be cosmetically undesirable. Such stresses are detrimental to the hair fiber, and often alter the hair fiber's mechanical properties such as the tensile strength, the breaking load and the elasticity, or its resistance to swelling in an aqueous medium.

There is a clear interest to preserve and/or enhance the mechanical properties of the hair, and especially the resistance of hair to break upon combing which is an indirect measure of the hair's tensile strength and elasticity, in relation to different types of damage caused by the various stressing factors.

The subject of the present invention is therefore a method of treating hair for protecting and/or enhancing its mechanical properties, and particularly resistance of hair to break upon combing.

Several approaches have been proposed to increase or maintain the hair's mechanical properties such as strength.

One approach is to protect the hair in order to minimize the impact of the stressing factors to which it will be subjected. In this line it has been proposed to use for example ultraviolet light filters to reduce the detrimental effect of light in hair (see for example G.B. Patent No 2 212 048 A1).

Another approach is to treat hair with compositions which improve the strength and/or other mechanical properties of hair which has already been damaged.

U.S. Patent 3,842,848 describes a method of bonding especially prepared hydrolyzed peptide products of keratinaceous materials to human hair. The process is effected by conducting the reducing step of permanent waving in the presence of the peptide products and, thereafter, in a second step, oxidizing.

EP 0 403 530 A1 describes the treatment of hair with compositions comprising proteins which have been activated by reducing their disulfide groups, together with reducing agents and oxidizing agents. Compositions which contain film-forming protein (preferably an activated thiol-containing film-forming protein) and a reducing agent tend to develop a pungent, malodor which in certain compositions may be quite unpleasant.

The amount of film-forming protein that binds to skin will vary greatly as a function of the type of film-forming protein that is used. Protein that does not contain sufficient cysteinyl thiol groups does not bind to the keratinous substrate to the same extent or for the same duration of time as does protein containing sufficient cysteinyl thiol groups.

Moreover, such a protein does not intercalate to become part of the natural skin as is the case of the protein keratin. In the case of activated protein, the percent of activated protein that bonds to the keratinous tissue will vary with the concentration of reducing agents in the composition and the number of activated thiol or mercapto groups in the activated protein and the keratinous tissue.

The above mentioned solutions require the use specially treated proteins with undesirable odour or the use of oxidizing and/or reducing agents to enhance the strength-increasing effect of the protein derivatives used.

### DETAILED DESCRIPTION OF THE INVENTION

After extensive research the applicants have now developed a process of treating hair to improve its resistance to break upon combing which involves the use of protein derivatives and achieves deep penetration of the proteins into the hair structure for optimum results.

Without willing to be bound by theory, it is hypothesized that the process according to the present invention allows at least part of the protein derivatives to penetrate the hair structure and reach the cortex of the hair. The cortex is composed of a series of fibers whose health and vitality determine the elasticity and strength of each hair. It consists mainly of clusters of cells elongated parallel filaments oriented in the direction of the hair fiber. These filaments or microfibrils are grouped into more complex organizations, the macrofibrils. The microfibrils contain alpha-keratin, a protein characteristic of the hair fiber, organized in the form of alpha-helix, which is what gives the hair its mechanical properties.

Thus, the process of the invention allows not only restructuring the outside of the hair (which produces a softer feel, conditioning, etc.) but also repairing the inside portion of the hair or cortex. The process of the invention provides particularly good results when a mixture is used comprising quaternized hydrolyzed keratin, hydrolyzed keratin and keratin amino acids.

In one aspect of the present invention provides a process for improving at least the strength of hair comprising the following steps:
i. applying to hair of a slightly alkaline aqueous composition having a pH between 7.5 and 9.0, preferably 7.5-8.5.
ii. at least partially removing the composition applied in step i)
iii. applying to hair a composition comprising quaternized hydrolyzed keratin, hydrolyzed keratin and keratin amino acids
iv. leaving on the composition applied in step iii) during 0.25 to 8 hours at a temperature comprised between 15 and 70°C
v. at least partially removing the composition applied in step iii)
vi. applying to wet hair a shampooing composition comprising a surfactant and having a (slightly acidic) pH not greater than 5.5, preferably between 4.5 and 5.5.
vii. substantially removing the composition applied in step vi)

In one embodiment of the present invention step ii) comprise the steps of:
a) rinsing hair, preferably with water or with water and a shampooing composition,
b) optionally reducing the hair's water content by applying heat, draining or contacting hair with a woven or non-woven textile having water-absorbing properties such as a towel

In another embodiment of the present invention steps i) and ii) are repeated one or more times.

In still another embodiment of the present invention hair is massaged after the application of the composition applied in step (iii) .

As described above the process of the invention involves applying to hair (in step (iii) a composition comprising quaternized hydrolyzed keratin, hydrolyzed keratin and keratin amino acids. The composition applied in step (iii) is to be left in contact with hair for a time period range from 0.25 to 8 hours at a temperature ranging from 15 to 70 °C. The time period during which the above mentioned composition is left in contact with hair is a function of the temperature at which hair is maintained during the contact time. In effect, when hair is maintained at ambient temperature (15 to 30 °C) it is normally necessary to keep the composition applied in step (iii) in contact with hair for a time period ranging from 6 to 8 hours. When hair is maintained at a high temperature comprised between 50 and 70°C it is normally sufficient to keep the composition applied in step (iii) in contact with hair for a much shorter time period ranging from 0.25 to 1 hour. Between these two extremes specific combinations of time and temperature can be selected taking into account than lower temperatures will require higher times of contact.

It has also proven to be advantageous although not necessary to wrap hair with a woven or non-woven textile having water-absorbing properties, specially when the composition applied in step (iii) is kept in contact with hair at ambient temperature (15 to 30°C).

Thus, in one embodiment of the present invention the composition applied in step iii) is left during 6 to 8 hours at a temperature comprised between 15 and 30°C, preferably after hair has been wrapping with a woven or non-woven textile having water-absorbing properties after application of such composition.

In still another embodiment of the present invention the composition applied in step iii) is left during 15 to 60 minutes while hair is maintained at a temperature comprised between 50 and 70°C

In still another embodiment of the present invention step v) comprises the steps of:
c) rinsing hair, preferably with water
d) optionally reducing the hair's water content by applying heat, draining or contacting hair with a woven or non-woven textile having water-absorbing material such as a towel

In still another embodiment of the present invention hair is massaged after the application of the shampooing composition in step vi).

In still another embodiment of the present invention step vii) comprises the steps of:
e) rinsing hair, preferably with water
f) substantially reducing the hair's water content by applying heat, draining or contacting hair with a woven or non-woven textile having water-absorbing properties such as a towel

In another embodiment of the present invention steps vi) and vii) are repeated one or more times.

In still another embodiment of the present invention a finishing composition comprising conditioning agent is applied after step vii.

In still another embodiment of the present invention the shampooing composition applied in step vi) comprises a surfactant. In a preferred embodiment the shampooing composition may also comprise quaternized hydrolyzed keratin.

In a preferred embodiment the present invention provides a process for improving at least the strength of hair comprising the following steps:
i. applying to hair of a slightly alkaline aqueous composition having a pH between 7.5-9.0, preferably 7.5-8.5.
ii. rinsing hair
iii. optionally repeating steps i and ii
iv. optionally reducing the hair's water content
v. applying to hair a composition comprising quaternized hydrolyzed keratin, hydrolyzed keratin and keratin amino acids
vi. optionally massaging hair
vii. optionally either applying heat for a period of time of between 15 and 60 minutes or alternatively wrapping hair with a woven or non-woven textile having water-absorbing properties
viii.leaving on the composition applied in step vii) during 6 to 8 hours
ix. optionally rinsing hair
x. applying to wet hair a shampooing composition comprising a surfactant and having a (slightly acidic) pH not greater than 5.5, preferably between 4.5 and 5.5,
xi. optionally massaging the hair and scalp
xii. rinsing hair
xiii.optionally repeating steps x to xii
xiv. optionally drying hair
xv. optionally applying a finishing composition comprising a conditioning agent.

In another aspect the present invention provides a kit comprising at least a) a slightly alkaline aqueous composition having a pH between 7.5 and 9.0, preferably between 7.5 and 8.5, b) a composition comprising quaternized hydrolyzed keratin, hydrolyzed keratin and keratin amino acids and c) a shampooing composition comprising a surfactant and having a (slightly acidic) pH not greater than 5.5, preferably between 4.5 and 5.5.

In another embodiment the present invention the kit comprises in addition to a) the slightly alkaline aqueous composition, b) the composition comprising keratin derivatives and c) the shampooing composition also a finishing composition d) .

The kit comprising the above mentioned products enables a treatment as described above specially designed for hair which is dry, rough, brittle, frizzy, malnourished, stiff and dull, and or has been mistreated by external conditions, either environmental or physical-chemical, which have been deteriorating hair until he lost his balance and natural beauty.

In one embodiment the present invention provides a kit comprising at least a) a slightly alkaline aqueous composition having a pH between 7.5 and 9.0, preferably between 7.5 and 8.5, b) a composition comprising quaternized hydrolyzed keratin, hydrolyzed keratin and keratin amino acids and c) a shampooing composition comprising a surfactant, quaternized hydrolyzed keratin, hydrolyzed keratin and keratin amino acids and having a (slightly acidic) pH not greater than 5.5, preferably between 4.5 and 5.5.

The term "at least partially removing" is used in the present application to designate an action that achieves a reduction of at least 50% by weight, preferably at least 70% by weight, more preferably at least 80% by weight and still more preferably at least 90% by weight of the component which is preceded by the expression.

The term "substantially removing" is used in the present application to designate an action that achieves a reduction of at least 70% by weight, more preferably at least 80% by weight and still more preferably at least 90% by weight of the component which is preceded by the expression.

The term "woven or non-woven textile having water-absorbing properties" is used in the present invention to designate materials which upon contact with wet hair are able to at least partially remove water therefrom.

The term "substantially reducing the hair's water content" is used in the present invention to designate an action that reduces hair's water content by at least 70% by weight, more preferably at least 80% by weight and still more preferably at least 90% by weight.

### COMPOSITION A/ FOR STEP i)

The composition applied in step i) of the process of the invention is an aqueous composition having a pH between 7.5 and 9.0 which opens the cuticle of hair preparing it for the application of the keratin derivatives. Normally a pH adjusting agent is used to set the composition at the desired pH value. Depending on the additional optional ingredients present in the formulation the pH will be adjusted with acids or with bases. Examples of such pH adjusting agents are weak organic acids such as citric acid, tartaric acid, lactic acid and malic acid, organic amines such as triethanolamine and inorganic salts such as sodium hydroxyde.

In an embodiment of the present invention the slightly alkaline composition comprises surfactants to clean hair thoroughly while opening the hair cuticle. Surfactants may also be incorporated into the composition to help create an emulsion and to solubilise insoluble components.

The surfactants may be selected from the group comprising a) anionic surfactants such as alkyl ether sulphates, alkyl sulphates, alkylbenzenesulfonates, sulphated alkanolamides, alfa olefinsulfonates and soaps, b) non-ionic surfactants such as ethoxylated alcohols, ethoxylated fatty acids, alkyl polyglucosides, ethylene oxide-propylene oxide copolymers, fatty acid mono- or dietanolamides, fatty alcohols and amine oxides, c) amphoteric or zwitterionic surfactants such as betaines, sulfobetaines, aminopropionic acids and imidopropionic acids .

Preferred surfactants are cocamide monoisopropanolamide, cocoamidopropylbetaine, decyl glucoside, lauryl glucoside, PEG-7 glyceryl cocoate, lauryl pyrrolidone, disodium cocoamphodiacetate, sodium laureth sulphate, cetearyl alcohol, glyceryl laurate, ethoxylated oleyl-cetyl alcohol (oleth-5), polyethylene glycol diester of stearic acid (PEG-150 distearate), ethoxylated/propoxylated tridecyl ether (PPG-1 trideceth-6), alkoxylated di-ester of myristyl alcohol and adipic acid (Di-PPG-2-myreth-10 adipate) and ethoxylated hydrogenated castor oil (PEG-60 hydrogenated castor oil).

The composition applied in step i) may also comprise antistatic agents such as polyquaternium-7, conditioning agents such as PEG-12 dimethicone, and panthenol, antimicrobial agents such as rosemary extracts and antioxidants such as salvia extracts, solvents such as 1,2-hexanediol, ethanol, propylene glycol, and water, chelating agent such as EDTA and its salts, emollients such as caprylyl glycol and preservatives such as phenoxyethanol.

The aesthetics look and feel of the composition may also be established using perfumes, colorants, pigments, viscosity adjusting agents and the like.

### COMPOSITION B/ FOR STEP iii)

The composition applied in step iii) of the process of the invention comprises a mixture of quaternized hydrolyzed keratin, hydrolyzed keratin and keratin amino acids. In one embodiment of the present invention the composition comprises also other antistatic agents such as dicetyldimonium chloride and polyquaternium-10, other conditioning agents such as dimethicone, dimethiconol, phenyl trimethicone and panthenol, surfactans such as PEG-150 distearate, chelating agents such as EDTA, perfumes and emollients such as cetearyl alcohol and C12-15 alkyl benzoates. In addition, preservatives such as diazolidinyl urea and phenoxyethanol, solvents such as isopropyl alcohol and water and viscosity increasing agents such as hydroxypropyl starch phosphate and hydroxyethyl cellulose may also be added to finish the composition.

### COMPOSITION C/ FOR STEP vi)

The composition applied in step vi) of the process of the invention is a shampooing composition comprising a surfactant such as, for example, cocoamidopropyl betaine, decyl glucoside, lauryl glucoside and sodium laureth sulphate and having a (slightly acidic) pH not greater than 5.5, preferably between 4.5 and 5.5 which closes the cuticle of hair trapping the keratin derivatives applied in step iii).

The composition applied in step iii) may also comprise antistatic agents such as polyquaternium-10, emulsifying agents such as PEG-7 glyceryl cocoate and glyceryl laurate, solubilising agents such as di-PPG-2-myreth-10 adipate, emollients such as caprylyl glycol, humectants such as sodium PCA, conditioning agents such as panthenol and ceramide 3, hair antistatic agents such as polyquaternium-7, cocodimonium hydroxypropyl hydrolyzed keratin, conditioning agents such as hydrolyzed keratin and keratin aminoacids , chelating agents suchs as EDTA, solvents such as 1,2-hexanediol and preservatives such as phenoxyethanol.

The aesthetic look and feel of the composition may also be established using perfumes, colorants, pigments, opacifying agents such as PEG-3 distearate, viscosity adjusting agents such as cocamide monoisopropylamine and the like.

### FINISHING COMPOSITION D/

The composition is designed to provide a glossy finish styling and natural glow.

The composition applied in step iii) may comprise conditioning agents such as panthenol, amodimethicone, cyclopentasiloxane, phenyltrimethicone, disiloxane, wheat aminoacids and dimethiconol, antistatic agents such as polyquaternium-11, polyquaternium-37 and hydroxypropyl guar hydroxypropyltrimonium chloride, emollients such as propylene glycol dicaprilate/ dicaprate, emulsifying agents such as PEG/PPG-18/18 dimethicone, oleth-5 and PPG-1 trideceth-6, hair fixatives such as polyquaternium-55, humectants such as mannitol, solvents such as ethanol, propylene glycol and water, chelating agents such as EDTA, preservatives such as diazoidinyl urea and methylisothiazolidone and agents for UV protection such as benzophenone.

The aesthetic look and feel of the composition may also be established using perfumes, colorants, pigments, opacifying agents, viscosity adjusting agents and the like.

### DETAILED DESCRIPTION OF THE INGREDIENTS

### ANTISTATIC AGENTS

The term "quaternized hydrolyzed keratin" is used in the present application to designate the products obtained from the reaction of hydrolyzed keratin with the epoxides derived from hydroxypropyl-C₁₋₂₂-alkyldimethylammonium salts under alkaline conditions as explained below.

Typically, keratin hydrolizates in liquid form are reacted in solution in a homogeneous system. Here, the substrate solution is mixed with the cationizing reagent and sufficient alkali to convert the chlorohydrin form into its reactive epoxide form (stoichiometric alkali) and to bring the pH up to the required alkalinity for the reaction to proceed (catalytic alkali). This solution is then maintained at elevated temperatures for up to 24 hours in order to completely consume the epoxide form of the reagent. Subsequently, the final mixture is neutralized with an acid to a neutral pH. In general, no further purification is performed. Tipically quaternized hydrolyzed keratin products are used in the form of cocodimonium hydroxypropyl hydrolized keratin and have an average molecular weight in the range of 500-5000 Da, preferably 800-1500 Da, more preferably 900-1100 Da.

Polyquaternium 7 (copolymer of acrylamide and diallyldimethylammonium chloride) is a highly charged cationic copolymer which is useful for improving the properties in wet and dry hair products. It provides excellent lubricity, ease of combing of wet hair and shine.

Polyquaternium 37 is the INCI name used for poly(2-methacryloxyethyltrimethylammonium chloride)a methacrylated polymer with quartenized pendant groups.

Quaternary ammonium salts such as dicetyldimonium chloride are positively charged so that they can be deposited on the surface of hair, which is negatively charged. Its excellent cosmetic properties leave hair easier to manage and impart excellent sensory qualities, packaging and handling.

Hydroxypropyl guar hydroxypropyltrimonium chloride is a quaternary ammonium derivative of hydroxypropyl guar.

Polyquaternium 22 is the INCI name used for the copolymer of acrylic acid and diallyldimethylammonium chloride and is a highly charged cationic copolymer suitable for use in products for dry, chemically abused or black hair and oriental. It provides excellent slip and lubricity. Increase the amount and the creaminess of the foam. It also improves wet comb and dry hair manageability.

Quaternium 91 (dibehenyl imidazolinium methosulfate) is an anti-static conditioner that conditions, detangles and softens hair in intensity. It presents very good properties of comb wet and dry.

Polyquaternium-10 (quaternized hydroxyethylcellulose) is a natural cellulosic polymer that forms on the surface of the hair, in particular, covering the damaged spots due to its cationic character.

Polyquaternium-11 (copolymer of vinylpyrrolidone and quaternized dimethylaminoethyl methacrylate) is a synthetic polymer that forms on the surface of the hair, in particular, covering the damaged spots due to its cationic character. That is why, they have a high substantivity and good comb and provide both wet and dry.

### CONDITIONING AGENTS

Amodimethicone is an abbreviation of "amine-functionalized silicone," designating a family of silicones which have been chemically modified so that some of the pendant groups along the backbone have been replaced with various alkylamine groups (-R-NH2). These amine groups become positively charged in aqueous solutions because of their electron-donating (basic) tendencies, yielding an inorganic, cationic polymer.

The term "hydrolyzed keratin" is used in the present application to designate mixtures of low molecular weight polypeptides prepared by digesting keratinous proteins with alkali optionally followed by concentration and spray drying. When used as such hydrolyzed keratin have tipically an average molecular weight in the range of 100-1000 Da, preferably 300-700 Da, more preferably 450-550 Da.

The term "keratin aminoacids" is used in the present application to designate the mixture of aminoacids resulting from the complete hydrolysis of keratinous proteins. Keratin is a fibrous protein composed of 18 different amino acids. Unlike collagen, which is produced by cells of the dermis, keratin is produced in the body by the epidermal cells and is the main protein in hair and nails. Keratin amino acids are obtained through hydrolysis of the protein chain.

Cyclopentasiloxane (2,2,4,4,6,6,8,8,10,10-decamethylcyclopentasiloxane) is a cyclic polysiloxane showing emollient properties.

Dimethicone is the original silicone is oily and difficult to extend. The viscosity of the silicone used in Extreme Makeover Kit is a low viscosity silicone, which has very well balanced properties. Provides elasticity, lubricity, softness and shine but it is neither voiceless nor engage and oily residue left on the hair.

Dimethiconol is a dimethyl siloxane terminated with hydroxyl groups which has properties of repair, especially for its cationic properties, which lead it to be placed at the ends where hair is usually more severely punished. It is also film-forming, thus providing, softness to the hair along its length.

Disiloxane: Is a polydimethylsiloxane (CH₃)₃-Si-(SiO₂)-Si(CH₃)₃ fluid with good spreading and volatility characteristics.

Panthenol or provitamin B5 is a precursor of vitamin B5 (unstable in cosmetics) is beneficial to the skin, hair and nails. Topical application accelerates cellular regeneration of the skin and allows it to remain hydrated and elastic. It is a moisturizer that acts like hydrating the hair, giving hair fiber coating gloss without grease, increasing the flexibility and manageability of the hair. It also helps to provide volume and fullness to hair.

PEG-12 dimethicone is a silicone glycol copolymer surfactant that acts to stabilize the foam shampoo. It also facilitates hair, improving manageability and providing a silky feel slightly conditioning.

Phenyl trimethicone is a phenyl substituted silicone fluid whose main benefit is to provide the hair shine and color protection.

Wheat aminoacids is a mixture of amino acids resulting from the complete hydrolysis of wheat proteins.

Ceramide 3 has a phytoshingosine backbone acylated with oleic acid. The constituent lipid ceramide is the most akin to the cuticle. It provides the cohesiveness of the scales of the cuticle, while avoiding internal dehydration. Therefore contributes to the repair and protection of damaged hair, providing comb and shine.

### SURFACTANTS:

Surfactants may be selected from the group comprising:
a) anionic surfactants such as alkyl ether sulphates, alkyl sulphates, alkylbenzenesulfonates, sulphated alkanolamides, alfa olefinsulfonates and soaps. Anionic surfactants are usually employed as cleansing and/or emulsifying agents.
b) non-ionic surfactants such as ethoxylated alcohols, ethoxylated fatty acids, alkyl polyglucosides, ethylene oxide-propylene oxide copolymers, fatty acid mono- or dietanolamides, fatty alcohols and amine oxides,
c) amphoteric or zwitterionic surfactants such as betaines, sulfobetaines, aminopropionic acids and imidopropionic acids.

Preferred anionic surfactants comprise sodium laureth sulphate which is frequently used as cleansing agent.

Preferred nonionic surfactants are decyl glucoside, lauryl glucoside, lauryl pyrrolidone, glyceryl laurate, oleth-5, and PEG/PPG-18/18 dimethicone, PEG-150 distearate, PEG-7 glyceryl cocoate, PPG-1-trideceth-6, cocamide MIPA, Di-PPG-2-myreth-10 adipate and PEG-60 hydrogenated castor oil.

Decyl glucoside and lauryl glucoside are mild non-ionic surfactants which are ethers from glucose and decyl alcohol and lauryl alcohol respectively and which are frequently used as cleansing agents.

Glyceryl laurate also known as glycerol monolaurate is a monoglyceride surfactant. It is the ester formed from glycerol and lauric acid and is an emollient and an emulsifying agent.

Oleth-5 is a mixture of ethoxylated oleyl and cetyl alcohols.

PEG-150-distearate is a polyethylene glycol diester of stearic acid is used as a thickener for surfactant-containing products, as a solubilizer for various water-insoluble ingredients and as a co-emulsifyer in creams & lotions.

PEG-7 glyceryl cocoate is an emollient oil that provides soft light hair, providing hair and helping to eliminate static electricity hair.

PPG-1 Trideceth-6 is an ether of polyoxypropylene, polyoxyethylene and tridecyl alcohol.

Preferred amphoteric surfactants are cocoamidopropyl betaine and disodium cocoamphodiacetate,

Cocoamidopropyl betaine or {[3-(dodecanoylamino)propyl] (dimethyl)ammonio}acetate is a zwitterionic surfactant frequently used as a mild cleansing agent.

Disodium cocoamphodiacetate **also known as** cocoamphocarboxyglycinate oxide is an amphoteric surfactant frequently used as a mild cleansing agent.

### CHELATING AGENTS

Disodium EDTA is the disodium salt of ethylenediaminetetraacetic acid.

### EMOLLIENTS

C12-15 alkyl benzoates are ester of C12-C15 alkanols and benzoic acid having a low viscosity, high polarity and acting as an emollients. They have excellent extensibility properties providing excellent lubrication and a light fitting without feeling oily or heavy.

Caprylyl glycol also known as 1,2-octanediol is an emollient with good humectants and antimicrobial properties.

Propylene Glycol Dicaprylate/Dicaprate is the mixed diester of propylene glycol and a blend of C8-C10 fatty acids and is an emollient ester which is compatible with anionic and non-ionic surfactants.

Cetearyl alcohol is a mixture of fatty alcohols, consisting predominantly of cetyl and stearyl alcohols. It is used as an emulsion stabilizer, opacifying agent, and foam boosting surfactant, as well as an aqueous and nonaqueous viscosity-increasing agent. It imparts an emollient feel to the skin and can be used in water-in-oil emulsions, oil-in-water emulsions, and anhydrous formulations. It is commonly used in hair conditioners and other hair products.

### HUMECTANTS

Sodium pyrrolidine carboxylate (Sodium PCA) or sodium 5-oxo-pyrrolidine-2-carboxylate is a natural humectant derived from L-Glutamic acid. It is a major component of natural moisturizing factor of the skin (FMN), helps keep skin and hair looks healthy and fresh. In hair, it also facilitates wet hair and reduces static electricity, providing an aspect of natural and healthy hair.

D-mannitol or 1,2,3,4,5,6-hexol is an humectant polyol.

### EMULSIFYING AGENTS

In addition to the non-ionic surfactants oleth-5, PEG-150 distearate, PPG-1 trideceth-6 and PEG-7 glyceryl cocoate which have been described above, PEG/PPG-18/18 dimethicone may also be used as an emulsifying agent.

PEG/PPG-18/18 dimethicone is the alkoxylated derivative of dimethicone containing an average of 18 moles of ethylene oxide and 18 moles of propylene oxide.

### OTHER INGREDIENTS

Benzophenones are broad-spectrum filters as they absorb the radiation UVA (320-400 nm) and UVB (280-320 nm). Scientific papers show that benzophenones are capable of protecting the structure and color of hair, preventing damage from UV radiation.

Salvia extract (Salvia Officinalis) are extracts from the leaves of Salvia officinalis containing active substances which makes it active antiseptic, antiphlogistic, fungicidal and antioxidant. In addition it has also been reported to tonify and strengthen the hair's root and to normalise sebum production.

Rosemary Extract (Rosmarinus Officinalis) are extracts from the leaves of the plant, which contain substances such as caffeic acid, rosmarinic acid and rutin, with antimicrobial properties which have also been reported to confer protective, invigorating and stimulating properties to hair and to normalize sebum production through its action on the enzyme 5-alpha-reductase.

### PRESERVATIVES:

Diazolidinyl urea also known as 1,3-bis(hydroxymethyl)-1-(1,3,4-tris(hydroxymethyl)-2,5-dioxoimidazolidin-4-yl)urea is and antimicrobial preservative widely used in cosmetics.

Methylisothiazolinone also known as 2-Methylisothiazol-3(2H)-one or MIT is a powerful biocide and preservative used in shampoos and body care products.

Phenoxyethanol also known as 2-phenoxy-l-ethanol is a bactericide used in dermatological products such as skin creams and sunscreens.

### HAIR FIXATIVES:

Polyquaternium 55 is a copolymer of N,N-Dimethyl-N-[3-(2-methyl-2-propenoyl)aminopropyl]-1-dodecanaminium chloride with N-(3-dimethylaminopropyl)-2-methyl-2-propenamide and 1-ethenyl-2-pyrrolidinone used as a hair fixative.

### OPACIFYING AGENTS:

PEG-3 distearate is the ester of stearic acid with a polyethyleneglycol having an average molecular weight of 186 Da.

### SOLVENTS:

Examples of solvents useful in the formulation of the compositions of the invention are 1,2-hexanediol, ethanol, isopropyl alcohol, propylene glycol, and water.

### VISCOSITY INCREASING AGENTS:

HYDROXYPROPYL STARCH PHOSPHATE: Hydroxypropyl Starch Phosphate is the hydroxypropyl ether of distarch phosphate which is in turn obtained by crosslinking starch with phosphate ester groups for example making use of phosphorous oxychloride or sodium trimetaphosphate. It is useful, for example, as a viscosity increasing agent..

Hydroxyethyl cellulose is a cellulose derivative wherein one or more of cellulose's free hydroxyl groups have been condensed with ethylene oxide or similar ethoxylating reagents. It is useful, for example, as a viscosity increasing agent.

Cocamide monoisopropanolamide also known as cocoamide MIPA designates the amide derived from coconut fatty acids and 2-hydroxypropylamine. It is useful, for example, as a viscosity increasing agent.

### PROTOCOL

The following protocol has been designed to measure the resistance of hair to break upon combing.

A method commonly used to make anti-breakage and protection claims for hair care products is the use of repeated combing operations. These experiments involve brushing and combing locks of hair and subsequently counting the number of broken fibers.

To this respect the test is made using a custom-built device described shown in Figure 2 and described in detail below:
This device is made up of a hollow assembly with a rotating drum, where four outer crosspieces contain supports for assembling combs or brushes. These outer arms are detachable and allow assembling different supports and use several types of brushes or combs for performing the experiments.

The four combs or brushes are assembled at 90° angles, allowing a complete revolution of the drum in order to comb a lock on four occasions. This device is duplicated on three occasions in a horizontal direction, allowing combing four braids simultaneously. The collector plates for storing broken fiber fragments are located under each lock, while the separation plates in the rotating drum prevent cross contamination. The device contains a variable-speed motor, although the experiments are generally performed at 50 brushes per minute.

The size and type of comb may be selected as a function of the type of hair. In this particular case standard-sized combs have been used, which have a distance between the times of the comb of comprised between 2 mm.

The environment of the device is maintained at a constant relative humidity of about 60%.

Hair locks are made of hair from a single Caucasian individual. The locks of hair weigh approximately 3 g, and are 20.23 cm long and 2.54 cm wide.

The locks are bleached twice using a 9% hydrogen peroxide solution with pH 10.2. The locks are left in contact with the solution for 40 minutes under controlled temperature conditions of 40°C. At the end of the process, the locks are rinsed under the tap at 40°C with a controlled flow of 3.78 1/min .

The bleached hair locks are the subjected to three different treatments described in the table below. Treatment A represents a conventional shampooing process while treatments B and C are two processes according to the present invention.

**Table 1**

| | **TREATMENT A** | **TREATMENT B** | **TREATMENT B** |
|---|---|---|---|
| **Step i** | Wash with 2% LESS solution | Wash with Composition A/ | Wash with Composition A/ |
| **Step ii** | Rinse 3 times with water for 1 minute each time. | Rinse | Rinse |
| **Step iii** | N/A* | Apply Composition B/ for 8 hours at 30°C and 100% RH | Apply Composition B/ for 0.5 hours at 60°C and 100% RH |
| **Step iv** | N/A | Wash with Composition C/ | Wash with Composition C/ |
| **Step v** | N/A | Rinse | Rinse |
| **Step vi** | Dry | Dry | Dry |
| **Step vii** | N/A | Apply Composition D/ | Apply Composition D/ |

| | | | |
|---|---|---|---|
| *N/A no product is applied | | | |

The concentration of Compositions A/ and C/ used in steps i) and iii) respectively is equal to 10% (0.3 g per 3g of lock). After wetting the lock, Composition A/ is applied performing a massage for 1 minute, followed by three rinsing cycles of one minute each.

Composition B/ is applied at 15% (0.45g per 3g of lock). The product is applied in the lock once it has partially dried with a towel. The lock is wrapped in a wet towel and introduced in the oven at the suitable temperature and for the desired time.

After washing the lock with Composition C/, it is rinsed and dried. Then, Composition D/ is applied at 15% (0.45g per 3g of lock) .

The entire rinsing is performed with a tap which uses water at 40°C with a flow of 3.78 1/min.

The treated locks are divided into ten equal portions which are then tested in the above mentioned apparatus. The number of broken fibers collected in the collector plates is counted after every 1000 combing operations until a total of 10000 combing operations..

**Table 2**

| | | **Number of broken fibers** | | |
|---|---|---|---|---|
| **Treatment** | **N** | **Mean** | **Std Dev** | **Std Err Mean** |
| A (Control) | 8 | 53.1 | 7.06 | 2.50 |
| B | 8 | 32.2 | 12.7 | 4.49 |
| C | 8 | 27.1 | 8.64 | 3.06 |

The table above shows the average number of broken hairs after combing stresses treated with treatments A, B and C a total of 10,000 combing operations. 8 stresses (N) of hair for each treatment are evaluated.

As will be apparent from the results of table 2 the processes of the present invention substantially improve the hair's mechanical properties, and particularly its resistance to breakage upon combing.

### EXAMPLES

**COMPOSITION A/ FOR STEP i)**

| INGREDIENTS | WT.% |
|---|---|
| CLEANSING AGENTS (SODIUM LAURETH SULFATE, DISODIUM COCOAMPHODIACETATE AND COCAMIDOPROPYL BETAINE) | 11.638 |
| SOLUBILIZING AGENT (PEG-60 HYDROGENATED CASTOR OIL) | 0.500 |
| EMULSIFYING AGENT (GLYCERYL LAURATE) | 0.140 |
| ANTISTATIC AGENT (POLYQUATERNIUM-7) | 0.045 |
| CONDITIONING AGENTS (PEG-12 DIMETHICONE AND PANTHENOL) | 0.550 |
| ANTIMICROBIAL (ROSMARINUS OFFICINALIS LEAF EXTRACT) | 0.014 |
| ANTIOXIDANT (SALVIA OFFICINALIS LEAF EXTRACT) | 0.004 |
| CHELATING AGENT (DISODIUM EDTA) | 0.100 |
| SURFACTANT (LAURYL PYRROLIDONE) | 0.750 |
| pH ADJUSTER (CITRIC ACID) | 0.080 |
| EMOLLIENT (CAPRYLYL GLYCOL) | 0.350 |
| PRESERVATIVE (PHENOXYETHANOL) | 0.800 |
| PERFUME | 0.684 |
| SOLVENTS (PROPYLENE GLYCOL, ETHANOL 96%,1,2-HEXANEDIOL AND WATER) | 84.336 |

| | |
|---|---|
| pH of Composition A = 7.8 | |

**COMPOSITION B/ FOR STEP iii)**

| INGREDIENTS | % W/W |
|---|---|
| ANTISTATIC AGENT (COCODIMONIUM HYDROXYPROPYL HYDROLYZED KERATIN) | 0.900 |
| CONDITIONING AGENT (HYDROLYZED KERATIN) | 5.000 |
| CONDITIONING AGENT (KERATIN AMINO ACIDS) | 0.675 |
| OTHER ANTISTATIC AGENTS (POLYQUATERNIUM-10 AND DICETYLDIMONIUM CHLORIDE) | 2.283 |
| OTHER CONDITIONING AGENTS (DIMETHICONOL, PANTHENOL, DIMETHICONE AND PHENYL TRIMETHICONE) | 3.020 |
| EMOLLIENTS (C12-15 ALKYL BENZOATE AND CETEARYL ALCOHOL) | 9.000 |
| VISCOSITY INCREASING AGENT (HYDROXYPROPYL STARCH PHOSPHATE AND HYDROXYETHYLCELLULOSE) | 3.156 |
| EMULSIFYING AGENT (PEG-150 DISTEARATE) | 0.150 |
| CHELATING AGENT (DISODIUM EDTA) | 0.100 |
| PRESERVATIVES (DIAZOLIDINYL UREA AND PHENOXYETHANOL) | 1.050 |
| PERFUME | 0.977 |
| SOLVENTS (ISOPROPYL ALCOHOL AND WATER) | 73.689 |

**COMPOSITION C/ FOR STEP vi)**

| INGREDIENTS | | % W/W |
|---|---|---|
| CLEANSING AGENTS | SODIUM LAURETH SULFATE | 9.100 |
| | COCAMIDOPROPYL BETAINE | 2.058 |
| | DECYL GLUCOSIDE | 0.795 |
| | LAURYL GLUCOSIDE | 0.005 |
| SOLUBILIZING AGENT (DI-PPG-2 MYRETH-10 ADIPATE) | | 3.000 |
| EMULSIFYING AGENT (PEG-7 GLYCERYL COCOATE AND GLYCERYL LAURATE) | | 1.210 |
| VISCOSITY INCREASING AGENT (COCAMIDE MIPA) | | 0.500 |
| ANTISTATIC AGENTS (COCODIMONIUM HYDROXYPROPYL HYDROLYZED KERATIN, POLYQUATERNIUM-7 AND POLYQUATERNIUM-10) | | 0.608 |
| CONDITIONING AGENTS (KERATIN AMINO ACIDS, HYDROLYZED KERATIN, PANTHENOL AND CERAMIDE 3) | | 0.714 |
| HUMECTANT (SODIUM PCA) | | 0.100 |
| EMOLLIENT (CAPRYLYL GLYCOL) | | 0.350 |
| OPACIFYING AGENT (PEG-3 DISTEARATE) | | 1.000 |
| CHELATING AGENT (DISODIUM EDTA) | | 0.100 |
| PRESERVATIVE (PHENOXYETHANOL) | | 0.800 |
| PERFUME | | 0.462 |
| SOLVENTS (1,2-HEXANEDIOL AND WATER) | | 79.198 |

| | | |
|---|---|---|
| pH of Composition C = 5.3 | | |

**FINISHING COMPOSITION D/**

| INGREDIENTS | % W/W |
|---|---|
| CONDITIONING AGENTS (DISILOXANE, PHENYL TRIMETHICONE, AMODIMETHICONE, CYCLOPENTASILOXANE, DIMETHICONOL, PANTHENOL AND WHEAT AMINOACIDS) | 3.758 |
| ANTISTATIC AGENTS (POLYQUATERNIUM-37, POLYQUATERNIUM-11 AND HYDROXYPROPYL GUAR HYDROXYPROPYLTRIMONIUM CHLORIDE) | 1.954 |
| EMULSIFYING AGENTS (PEG/PPG-18/18 DIMETHICONE, PPG-1 TRIDECETH-6 AND OLETH-5) | 0.530 |
| EMOLLIENT (PROPYLENE GLYCOL DICAPRYLATE/DICAPRATE) | 1.365 |
| HAIR FIXATIVE (POLYQUATERNIUM-55) | 0.100 |
| HUMECTANT (MANNITOL) | 0.050 |
| CHELATING AGENT (DISODIUM EDTA) | 0.100 |
| PRESERVATIVES (DIAZOLIDINYL UREA AND METHYLISOTHIAZOLINONE) | 0.207 |
| COLORANT (PIGMENTS) | 0.500 |
| PERFUME | 0.597 |
| SOLVENTS (PROPYLENE GLYCOL, ALCOHOL DENAT. AND WATER) | 90.839 |

## Claims

1. A process for improving resistance of hair to break upon combing comprising the following steps:
i. applying to hair of a slightly alkaline aqueous composition having a pH between 7.5 and 9.0, preferably 7.5 and 8.5.
ii. at least partially removing the composition applied in step i)
iii. applying to hair a composition comprising quaternized hydrolyzed keratin, hydrolyzed keratin and keratin amino acids
iv. leaving on the composition applied in step iii) for a time period ranging from 0.25 to 8 hours at a temperature ranging from 15 to 70 °C
v. at least partially removing the composition applied in step iii)
vi. applying to wet hair a shampooing composition comprising a surfactant and having a (slightly acidic) pH not greater than 5.5, preferably between 4.5 and 5.5,
vii. substantially removing the composition applied in step vi)

2. A process according to claim 1 wherein step ii) comprise the steps of:
a) rinsing hair, preferably with water or with water and a shampooing composition,
b) optionally reducing the hair's water content by applying heat, draining or contacting hair with a woven or non-woven textile having water-absorbing properties such as a towel

3. A process according to anyone of claims 1 and 2 wherein steps i) and ii) are repeated one or more times.

4. A process according to anyone of claims 1 to 3 wherein hair is massaged after step iii).

5. A process according to anyone of claims 1 to 4 wherein in step (iv) hair is kept at a temperature ranging from 15 to 30 °C for a time period ranging from 6 to 8 hours.

6. A process according to anyone of claims 1 to 4 wherein in step (iv) hair is kept at a temperature ranging from 50 to 70°C for a time period ranging from 0.25 to 1 hour.

7. A process according to anyone of claims 5 and 6 wherein hair is wrapped with a woven or non-woven textile having water-absorbing properties after step (iii) and un-wrapped after step (iv) .

8. A process according to anyone of claims 1 to 7 wherein step v) comprises the steps of:
c) rinsing hair, preferably with water
d) optionally reducing the hair's water content by applying heat, draining or contacting hair with a woven or non-woven textile having water-absorbing material such as a towel

9. A process according to anyone of claims 1 to 8 wherein hair is massaged after the application of the shampooing composition in step vi).

10. A process according to anyone of claims 1 to 9 wherein step vii) comprises the steps of:
e) rinsing hair, preferably with water
f) substantially reducing the hair's water content by applying heat, draining or contacting hair with a woven or non-woven textile having water-absorbing properties such as a towel

11. A process according to anyone of claims 1 to 10 wherein steps vi) and vii) are repeated one or more times.

12. A process according to anyone of claims 1 to 11 wherein a finishing composition comprising a conditioning agent is applied after step vii).

13. A process according to anyone of claims 1 to 12 wherein the shampooing composition applied in step vi) comprises a surfactant, quaternized hydrolyzed keratin, hydrolyzed keratin and keratin amino acids.

14. A process according to anyone of claims 1 to 13 comprising the following steps:
i. applying to hair of a slightly alkaline aqueous composition having a pH between 7.5-9.0, preferably 7.5-8.5.
ii. rinsing hair
iii. optionally repeating steps i and ii
iv. optionally reducing the hair's water content
v. applying to hair a composition comprising quaternized hydrolyzed keratin, hydrolyzed keratin and keratin amino acids
vi. optionally massaging hair
vii. optionally wrapping hair with a woven or non-woven textile having water-absorbing properties
viii.leaving on the composition applied in step vii) for a time period ranging from 0.25 to 8 hours at a temperature ranging from 15 to 70 °C
ix. optionally rinsing hair
x. applying to wet hair a shampooing composition comprising a surfactant and having a (slightly acidic) pH not greater than 5.5, preferably between 4.5 and 5.5,
xi. optionally massaging the hair and scalp
xii. rinsing hair
xiii.optionally repeating steps x to xii
xiv. optionally drying hair
xv. optionally applying a finishing composition comprising a conditioning agent.

15. A kit to carry out the process defined in anyone of claims 1 to 14 comprising at least:
a) a slightly alkaline aqueous composition having a pH between 7.5 and 9.0, preferably between 7.5 and 8.5,
b) a composition comprising quaternized hydrolyzed keratin, hydrolyzed keratin and keratin amino acids; and
c) a shampooing composition comprising a surfactant and having a (slightly acidic) pH not greater than 5.5, preferably between 4.5 and 5.5.

16. A kit according to claim 15 additionally comprising finishing composition d)
